# EUROPEAN PATENT APPLICATION

(11) **EP 4 803 107 A2**
(43) Date of publication of application: **09.09.2026**
(21) Application number: 26183165.5
(22) Date of filing: 02.05.2024
(51) Int. Cl.: A61L 2/10, A61L 2/00, A61L 2/02, A61Q 5/00, A61N 5/06, A61Q 19/00, A45D 40/00, A45D 19/00, A61L 2/025

(54) **DEVICE FOR TREATING HUMAN KERATINOUS MATERIAL INCLUDING AT LEAST ONE SOURCE OF UV RADIATION**

(30) Priority: 04.05.2023 FR 2304493; 04.05.2023 FR 2304494
(62) Document number(s) of the earlier application(s) in accordance with Art. 76 EPC: 24723833.0 / 4 704 925
(71) Applicant: L'OREAL, 75008 Paris (FR)
(72) Inventor: TOUCHARD, Violette, 94152 CHEVILLY LARUE (FR); BORDEAUX, Dominique, 94152 CHEVILLY LARUE (FR)
(74) Representative: Ipsilon

(57) **Abstract**

Device (1) for treating human keratinous material (K), notably the skin and/or the hair, comprising:
- a treatment head (5) to be placed in contact with the keratinous material (K) that is to be treated, defining at least one treatment zone,
- at least one reservoir (7) for containing a fluid, notably a cosmetic composition (C) used in the treatment, this reservoir (7) having an internal surface in contact with the fluid,
- at least one source (20) of UV radiation,
- at least one fluid circulation circuit (6) connecting the reservoir (7) and the treatment zone, at least part of the internal surface of the reservoir (7) and/or of the fluid circulation circuit (6) being exposed to the UV radiation emitted by the source (20),

## Description

### Technical field

The present invention relates to devices for treating human keratinous material, and is more particularly concerned with those devices that have a cleaning and/or decontamination function.

### Prior art

The wavelength of category-C ultraviolet radiation (UV-C) ranges from 100 nm to 280 nm. UV-C is recognised as being effective in destroying or inactivating microorganisms by making structural changes to nucleic acids. This property is used for disinfection in a large number of applications.

Patent FR2918854 describes a method for decontaminating an applicator and/or a cosmetic product contained in a container by exposure to UV-C radiation.

Application WO2018046849 describes a decontamination device comprising a body housing a UV source, the body being intended to receive, removably, part of a device used for packaging and dispensing a cosmetic product.

Patent US7560706 describes a germicidal device comprising a UV source for irradiating cosmetic compositions and cosmetic utensils that are to be sterilised.

Patent US9006680 describes a UV-impermeable cap enclosing a volume of fluid and comprising a source of UV radiation that irradiates the inside of the fluid volume.

Patents US11214499 and US10988389 describe stoppers for disinfecting water, notably drinking water, to cover a bottle, the stopper comprising a source of UV radiation.

Application US6086760 describes a UV steriliser assembly intended to be used in aquariums, the sterilising assembly having a vertically-oriented tubular housing which forms a sterilising chamber.

Application US5664340 describes a tumble dryer comprising, within the door, an antibacterial and antifungal UV bulb.

Patent US8414839 describes a system comprising a UV source for generating ozone in the water circulation pipes of spas, swimming pools, fountains, etc.

The applicant company has developed devices for the treatment of human keratinous material. These devices have the special feature of using at least one reservoir containing a fluid subjected to the action of ultrasound waves and a circuit for circulating this fluid, between the reservoir and a treatment zone and vice versa, and between various internal parts of the devices.

There is a need to be able to clean and disinfect such devices, notably the internal parts thereof, in a way that is effective and simple.

Moreover, there is a need to perform this cleaning and this disinfection without risk to or impact on the environment.

### Summary of the invention

The invention aims to meet these needs in full or in part and relates, according to a first of its aspects, to a device for treating human keratinous material, notably the skin and/or the hair, comprising:
- a treatment head to be placed in contact with the keratinous material that is to be treated, defining at least one treatment zone,
- at least one reservoir for containing a fluid, notably a cosmetic composition used in the treatment, this reservoir having an internal surface in contact with the fluid,
- preferably at least one ultrasound transducer for emitting acoustic waves at least into the fluid present in the treatment zone,
- at least one source of UV radiation,
- at least one fluid circulation circuit connecting the reservoir and the treatment zone, at least part of the internal surface of the reservoir and/or of the fluid circulation circuit being exposed to the UV radiation emitted by the source.

Another subject of the invention, according to a second of the aspects of this invention, independently of or in combination with the first, is a device for treating human keratinous material, notably the skin and/or the hair, comprising a handpiece comprising:
- a treatment head to be placed in contact with the keratinous material that is to be treated, defining at least one treatment zone,
- at least one reservoir for containing a fluid, notably a cosmetic composition used in the treatment,
- at least one circuit for the circulation of the fluid used in the treatment, comprising at least one canal for conveying the fluid to the treatment zone from the reservoir and at least one canal for returning the fluid from the treatment zone,
- preferably at least one ultrasound transducer for emitting acoustic waves at least into the fluid present in the treatment zone,
the device further comprising:
- a shut-off part intended to be brought into contact with the treatment head in the absence of use of the device for treating the human keratinous material, this shut-off part having a surface designed to close off the treatment zone in such a way as to allow the fluid to recirculate in the fluid circulation circuit without the treatment head being in contact with the human keratinous material.

The treatment of keratinous material using the device according to the invention may notably be a cleansing of the skin or of the hair, an exfoliation of the skin, the diffusion of active ingredients into the keratinous material and/or the treatment of certain skin disorders.

The fact that at least part of the internal surface of the reservoir and/or of the circulation circuit is exposed to the UV radiation emitted by the source according to the first aspect of the invention enables the risk of the multiplication of microorganisms within the fluid used for the treatment to be combated effectively, providing a disinfecting or decontaminating effect.

The use of UV radiation for cleaning and disinfecting is environmentally friendly and enables the use of chemical treatment agents to be avoided, or limited in terms of the quantity of agent used.

In addition, UV radiation does not result in any change, detrimental to the use of the device, in odour, in pH, in conductivity or in the chemical properties of the environment in which it is used.

The terms "disinfection" and "decontamination" are synonymous and mean that the microorganisms are destroyed by the UV radiation. It is possible for the decontamination of the surfaces or volumes exposed to the UV radiation not to be total, the level of destruction of the microorganisms preferably being greater than or equal to 90%, and even better, greater than or equal to 99.999%.

### Reservoir

The device comprises at least one reservoir for containing said fluid.

What should be understood by "reservoir" is any element able to contain the fluid; thus, the reservoir may be formed by any type of cavity, by a pouch or a cartridge, namely in this last case by an element that is removable.

The UV radiation source may be arranged so that it emits its UV radiation into said reservoir.

The device may comprise at least one UV radiation source, notably situated inside the reservoir.

The reservoir may even be exposed to UV radiation emitted by a source external to the reservoir. In that case, the source may be arranged so that it emits its radiation at least partially towards the opening of the reservoir, so that this radiation can reach the fluid contained in the reservoir and/or a surface of the reservoir that is in contact with the fluid.

The material of the reservoir may thus be chosen to be sufficiently transparent to the UV radiation so as to allow the UV to act through its wall or, as a variant, the reservoir is produced with a window that is transparent to the UV radiation emitted by the source.

The device may comprise several separate reservoirs, for example one reservoir of fluid to be distributed to the zone that is to be treated and one reservoir for containing used fluid that has been at least partially recovered following treatment. As a variant, the fluid coming from the reservoir is not recycled by the device or is sent back to the same reservoir from which it came, for example to an opposite end of this reservoir to the end from which it is taken.

The reservoir or reservoirs may or may not be removable. The reservoir or reservoirs are for example single-use reservoirs and may be replaced when there is no longer any fluid, or when the fluid recovered needs to be removed.

It is even possible to provide reservoirs the capacity of which varies according to the treatment to be performed on the keratinous material, it being possible for the device to be configured so as to allow the user to fit the reservoir of their choice, selected from among several with different respective capacities.

The reservoir may remain in the same place in the device when exposed to the UV radiation and when the device is being used to treat the human keratinous material.

As a variant, the reservoir is detached from the fluid circuit during the cleaning and/or decontamination phase, for example by being installed on a handpiece support, as will be described later on. In that case, the support may comprise at least one UV radiation source for decontaminating at least part of the reservoir.

When the device comprises a pump for circulating the fluid towards the treatment zone, the device advantageously, in order to facilitate the cleaning and/or decontamination of the reservoir, comprises a selector that allows the circulation of the fluid to be switched over from a first configuration in which circulation is between the reservoir or reservoirs and the treatment zone, and a second configuration used for purging and/or filling the reservoir or reservoirs. The inside of the pump may be exposed to the radiation from the UV radiation source or sources of the device and/or to the fluid previously decontaminated by the UV radiation and circulating through this pump.

The reservoir or reservoirs may comprise one or more compartments, notably several compartments integrated into the one same body and separated by a wall, or several communicating compartments, for example connected by a canal. The reservoir or reservoirs may comprise a compartment containing the fluid to be distributed to the zone that is to be treated, and a compartment collecting the used fluid, for example so that it can be recycled. The two compartments may communicate, for example via a filter.

The reservoir or reservoirs may even comprise several compartments in which different compositions may be stored. These compositions may be mixed at the time of use and may, for some of them, be solid and/or in the form of powders that are rapidly soluble.

For example, one reservoir may comprise a first compartment containing a solid composition, for example in the form of powder or crystals, and a second compartment comprising a fluid composition, it being possible for the two compartments to communicate with one another during use so that the fluid composition dissolves the solid composition.

The first compartment may even comprise a mixture of a solid composition in suspension and of a liquid in which the solid composition is insoluble, the fluid composition of the second compartment dissolving the mixture at the time of use of the device.

The reservoir or reservoirs may comprise one or more filters able to filter out impurities in the collected fluid before the fluid is redistributed to the zone that is to be treated. The reservoir may even comprise a vent.

The reservoir or reservoirs may comprise a device for heating or cooling the fluid, notably a resistive electrical element.

The reservoir or reservoirs are preferably compatible with one or more liquids of varying natures, notably different types of compositions, which may or may not be miscible, and cleaning liquids.

The reservoir or reservoirs are preferably at least partially transparent, thus enabling the user to make a visual assessment of their fill levels and/or of the level of cleanliness of the fluids that they contain.

The reservoir or reservoirs may be deformable or made of rigid material, notably of glass or plastic. A reservoir may comprise a removable part, and thus be of a volume that can be adjusted as desired. The reservoir or reservoirs may be fitted with check valves allowing them to be uncoupled from a receiving housing without loss of fluid.

The reservoir or reservoirs may take the form of cartridges of generally cylindrical or some other shape, or of flexible pouches.

At least one reservoir has a capacity for example of between 1 and 200 ml, better still between 1 and 50 ml.

The reservoir or reservoirs may comprise one or more fluid nonreturn devices, for example at least one check valve which opens automatically when the reservoir is fitted into a corresponding housing on the handpiece or its support, and which closes automatically when the reservoir has been detached from the handpiece or from the support, in order to avoid loss of liquid.

### Fluid circulation circuit

The fluid circulation circuit makes it possible to save water and/or fluid, notably to save cosmetic composition.

The fluid circulation circuit typically comprises a plurality of ducts, which are connected in an appropriate way for creating the desired circulation of fluid, for example with series or parallel connections, being joined together using appropriate connections.

The fluid circulation circuit may comprise at least an electric pump for circulating the fluid in the fluid circulation circuit, notably between the reservoir or reservoirs and the treatment zone.

The fluid circulation circuit connects together the various parts of the device that are involved with the circulation of the fluid, such as the treatment head, the pump or pumps and the fluid reservoir or reservoirs, as well as the filter or filters or other purification or disinfection systems or even additional bubble-generating systems.

The fluid circulation circuit may comprise one or more modifiable routings or valves, for example one or more manually operated or electrically controlled valves.

The fluid circulation circuit may comprise a water intake enabling it to be filled with water and/or cleaned from an external network.

The fluid circulation circuit may even be modifiable, with removable sections, for example sections that are added when the circuit is being cleaned. It is possible for example to add a section containing a cleaning powder which at least partially dissolves as a liquid is passed through the circuit. The section that is added in this way can be used only for cleaning, and then removed afterwards, or can be left in place until the next cleaning operation, whereupon it is then replaced with a new one.

The fluid circulation circuit may comprise at least one inlet and/or at least one outlet that can be connected to one or more secondary fluid-circulation circuits. The fluid circulation circuit may also be connected to one or more secondary reservoirs, notably containing different compositions from one another, this for example enabling the circulation of compositions in all forms as described hereinabove.

The reservoir or reservoirs and the fluid circulation circuit enabling the circulation of the fluid may belong to one and the same assembly forming a refill, this being able to be handled in one piece in order to be fitted on the device and removed therefrom; refilling with product is rendered easier as a result. Such a refill may, where appropriate, have a mechanical interface with the pump or other drive mechanism making it possible, when actuated, to establish a circulation of product from the refill towards the treatment zone.

The assembly that forms a refill may be designed to be fixed, for example by clip-fastening or in some other way, in the device, for example in one and the same casing as the one containing another component of the device, for example an electric generator.

The fluid circulation circuit may comprise at least one UV radiation source, notably situated inside the circuit, or outside but arranged such that the radiation reaches an internal surface of the circulation circuit in contact with the fluid.

Thus, at least one duct may be formed in a UV-transparent material, notably UVC-transparent material, the duct being situated for example close to the source.

### Treatment head

The treatment head is preferably designed to distribute the fluid to the zone that is to be treated via at least one fluid outlet, and/or to at least partially recover the used fluid from the zone that is to be treated using for example a fluid return inlet.

The outlet and/or the inlet are, for example, connected to the aforementioned fluid circulation circuit. In particular, the fluid circulation circuit may be arranged with respect to the treatment head in such a way as to partially distribute and/or collect the fluid in front of and/or behind the emission surface of the sonotrode of the transducer, for example through at least one orifice on the emission surface.

As a preference, the transducer extends at least partially into the treatment head. It may notably be fully housed therein.

The treatment head may comprise a heating element such as a resistive electrical element, for heating the composition for example, and/or a light source arranged to illuminate the treatment zone during use and/or to activate certain constituents of the fluid in which cavitation occurs.

The treatment head may comprise at least one filter, used for example for filtering the composition which is recycled, this filter being for example arranged in the return path of the fluid.

The treatment head may comprise a porous material and/or a material that is able to release or diffuse a fluid, notably an open-cell foam, preferably borne by a removable support, notably in the form of a frame.

The treatment head may comprise distribution orifices such as slots, which are closed at rest and open under the pressure of the composition upstream, and have an elasticity that allows an increase in volume during filling and distribution of fluid that continues after the end of the filling action.

The treatment head may comprise a chamber for storing a sufficient quantity of fluid to allow the cleansing of the keratinous material and the contact of the bubbles with the surface to be cleansed.

The treatment head may comprise several compartments which are either mutually independent or communicate with one another.

The treatment head may comprise parts made of a flexible and/or deformable material, notably an elastically deformable material, particularly as regards the parts in contact with the keratinous material that is to be treated.

The treatment head may have diverse shapes dependent on the intended use of the device.

The treatment head may comprise one or more removable parts that the user can easily detach from the device, preferably without resorting to any tool.

That makes cleaning between uses easier.

The removable part or parts of the treatment head may be attached to the rest of the device via any suitable fixing means, particularly a ball joint, a spring or a piston. The treatment head is preferably mobile; in certain examples it may be inclined or rotated through more than 180°, 270° or 360° about at least one axis.

The transducer is preferably arranged in relation to the treatment head in such a way as to keep the acoustic wave emission surface of the sonotrode away from the surface of the keratinous material that is to be treated.

Where applicable, the transducer is mobile relative to a fixed part of the device, for example driven in a rotating or back and forth movement.

Cavitation thus occurs in the front part of the head, in a space created between the emission surface of the sonotrode and the surface of the keratinous material that is to be treated.

The fluid may thus be made to circulate in this space, which is potentially modifiable.

This circulation may take place continuously, or intermittently. Said space may even be filled with the fluid while the handpiece is in place, the acoustic waves then being generated within the fluid filling this space.

The treatment head may comprise an endpiece, preferably removable, in contact with the keratinous material that is to be treated.

The endpiece, when removable, is for example chosen according to the use, for example by selecting the type, size, firmness and/or shape best suited to the morphology of the zone that is to be cleansed.

The treatment head may comprise a UV-radiation, notably UVC-radiation, source which is activated only during a phase of cleaning or decontamination of the device, and not while said device is being used for treating the keratinous material. This UV radiation source may be inside or outside the treatment head.

The treatment head may be detachable from the rest of the device.

### Shut-off part

The device preferably comprises a shut-off part intended to be brought into contact with the treatment head when the device is not being used for treating human keratinous material.

The shut-off part preferably has a surface designed to close off the treatment zone in such a way as to allow the fluid to recirculate in the fluid circulation circuit without the treatment head being in contact with the human keratinous material. In this way it becomes possible to circulate the fluid during the cleaning phase, during which the UV radiation source is activated. It is thus possible to expose a greater quantity of fluid to the UV radiation, and to benefit from the mechanical cleaning action exerted by the circulation of the fluid.

This may also allow the ultrasound generator to be brought into operation during the cleaning action, in order to improve same.

Of the shut-off part and the treatment head, at least one may bear a sealing gasket or sealing lip, so as to avoid loss of liquid via the treatment zone.

In particular, the device may comprise a flexible lip which serves to make a seal around the treatment head when the device is being used for treating the keratinous material, as detailed later on, and this flexible lip may serve to make a seal against the shut-off part.

The shut-off part may take the form of a removable closure cap or of part of a support cradle, as described later on.

### Handpiece

The device may comprise a handpiece, namely a piece the size of which allows it to be manipulated by the user in just one hand. The handpiece may comprise a surface forming a grip.

The handpiece may comprise the treatment head, the reservoir or reservoirs, the ultrasound transducer or transducers and/or the fluid circulation circuit.

The handpiece may comprise a guiding and/or combing member, an endpiece, a grille, one or more spacers and/or a flexible lip.

The handpiece may comprise at least an electric pump for circulating the fluid in the fluid circulation circuit between the reservoir or reservoirs and the keratinous material that is to be treated.

The handpiece can be manipulated in all orientations; it can be manipulated head up or head down for example.

At least one UV radiation source may be situated inside the handpiece, so as to expose to the UV radiation at least an internal surface of the reservoir or part of the fluid circulation circuit, and/or a surface of the treatment head in contact with the fluid.

The reservoir or reservoirs and/or the treatment head may or may not be detachable from the handpiece.

As a preference, the handpiece comprises a handle which can be arranged so that it is not coaxial with the treatment head. The handle may at least partially contain the fluid circulation circuit.

The reservoir or reservoirs may be arranged in the handpiece, notably in the handle thereof, or at least partially outside the handpiece. That may make them easier to detach, notably for positioning on a handpiece support cradle, which supports the handpiece when it is not in use.

The handpiece is preferably powered by an electric battery, but may also be connected to a power cord. The battery can be recharged directly by a power cord or by a support on which the handpiece is set down.

### Reflection/transmission of the UV radiation

The device, notably the handpiece, may comprise a material that reflects over 50% of the UV, notably UVC, radiation.

The reflective material is advantageously a metal, preferably aluminium or an aluminium alloy, although the invention is not restricted to such material.

The aluminium may be polished and reflect between 80% and 90% of the UV radiation.

At least part of an internal or external surface of the device may be covered with a UV-reflective, notably UVC-reflective, material, notably a metal coating, better still an aluminium coating, to enable the propagation of the UV radiation towards the surface or surfaces to be decontaminated.

At least part of the internal and/or external surface of the reservoir may be covered with a UV-reflective, notably UVC-reflective, material, notably a metal coating, better still an aluminium coating, notably so as to improve the action of the UV radiation in the reservoir.

At least part of a duct of the fluid circulation circuit may be covered with a metal reflective material, notably a metal coating, better still an aluminium coating, to encourage propagation of the UV radiation within the fluid circulation circuit and/or towards the reservoir. The fluid circulation circuit may be at least partially, or better still entirely, covered on its interior surface and/or on its exterior surface, with a reflective metal.

The device may comprise at least one material exposed to said UV radiation and transmitting over 80% of the UVC radiation, notably a material selected from the following list: FEP (fluorinated ethylene propylene) plastic, quartz, Plexiglass^{®} and/or a laminated glass with ionoplast interlayer. The device may more generally comprise any material capable of transmitting UV radiation.

FEP is able to withstand physical deformations and stresses such as creep, tensile stress and wear, while at the same time being resistant to cracking under stress.

### Support

The device may comprise a support designed to accept the handpiece when the device is not being used for the treatment of the keratinous material. This support may be in the form of a cradle on which the handpiece is set down when not being used for the treatment of the keratinous material.

The support may enable the recharging of a battery in the handpiece, used for powering the electronic circuit that ensures the operation thereof, and/or what may allow the handpiece to be put away while notably protecting the treatment head from becoming soiled.

The support may be designed to store internal and/or external parts of the handpiece and/or accessories, notably a brush, a spare head, a new reservoir, and/or cartridges of ancillary products.

The support may at least partially contain the fluid circulation circuit. In other words, there may be a fluidic connection between the handpiece and the support, enabling the fluid to be circulated through the support, notably with a view to exposing it to the UV radiation of a UV source contained in the support.

The support may be designed to electrically power the handpiece, notably the pump or pumps comprised within the handpiece, when the handpiece is held in the support. The handpiece can be charged, notably by the support, when the treatment head and/or the reservoir are detached therefrom.

The support may comprise a transformer or a converter connected to the electrical network.

The support and the handpiece may have electrical contacts that come into contact with one another when the handpiece is held on the support. As a variant, power is transmitted using induction.

The support may comprise a housing designed to accept the reservoir detached from the handpiece, notably designed to accept the reservoir with an opening of the reservoir being directed downwards. That may encourage the draining of the reservoir and allow the opening of the reservoir to be subjected to the UV radiation emitted by a source carried by the support, in front of which the opening is placed.

The aforementioned shut-off part may be defined by part of the support. That means that when the handpiece is placed back on the support, the latter shuts off the treatment head and allows fluid to be circulated through the treatment head without loss of fluid. This shut-off part may be detachable from the support, thereby allowing the shut-off part to remain in contact with the treatment head.

The support may electrically power the UV radiation source notably while the handpiece is present on the support and powered thereby. This is highly advantageous because it allows the handpiece to be operated from an external power supply rather than its internal battery, or allows the internal battery to be recharged at the same time as the handpiece is being used, such operation being aimed at cleaning and decontaminating the handpiece. This then means that more electrical power is available for simultaneously operating one or more UV sources that may be built into the handpiece, a circulation pump built into the handpiece and preferably also the ultrasound generator. The cavitation of bubbles during cleaning may promote the action of an active cleaning and/or decontamination agent, as well as the action of the UV.

The support may comprise at least one UV radiation source, notably one or two. This is advantageous because it makes it possible to avoid the need to incorporate the UV source into the handpiece, thus limiting the weight and complexity of the latter. In addition, it means that electrical power can be supplied more easily and that a higher power of UV radiation can be employed.

The device, particularly the support, may comprise at least one UV radiation source arrange to emit UV radiation inside the reservoir. In this case, it may be advantageous for the reservoir to be able to be detached from the handpiece and placed in a housing belonging to the support that enables it to be exposed to the UV radiation. The support may comprise a sensor that prevents the UV source from being operated in the absence of a reservoir in the dedicated housing for the support, so as to avoid exposing the user to the UV radiation. This sensor is, for example, an optical detector or a mechanical feeler or a magnetic sensor, the reservoir then being fitted with a corresponding activation magnet.

The support may comprise at least one UV radiation source arranged to emit UV radiation onto the treatment head.

In this case, the support may comprise a sensor that prevents the UV source from being operated in the absence of the handpiece on the support, so as to avoid exposing the user to the UV radiation.

When the reservoir is detachable from the handpiece, it is advantageous for the support to comprise a housing in which to place the reservoir, notably with its opening oriented downwards as mentioned above, and for the support also to allow the handpiece to be set down thereon both when the reservoir is in place on the handpiece and when the reservoir has been detached from the handpiece and installed in the corresponding housing belonging to the support.

### Flexible lip

The treatment head may comprise at least one flexible lip which may also act as a spacer in order to maintain the above-mentioned separation between the acoustic wave emission surface of the transducer and the surface that is to be treated.

The flexible lip, when the treatment head is applied to the keratinous material that is to be treated, may contribute to confining the fluid to the treatment zone.

The lip may be made up of sub-lips between which the fluid can be recovered.

The flexible lip is preferably made from a material containing silicone, polyurethane (PU), a natural or synthetic rubber, plastic, polyethylene terephthalate (PET) or else polythiophene (PT), or a combination of a number of these elements.

The flexible lip may be produced with reliefs or from a material that is intrinsically rough, so that it has a non-smooth surface for contact with the keratinous material. The flexible lip may thus contribute to the cleansing of the keratinous material thanks to a mechanical abrasive effect obtained when the device is moved over the zone that is to be cleansed while the device is in use.

The flexible lip may also contribute towards dispensing, spreading, collecting and/or recycling the fluid.

The flexible lip may be arranged in such a way as to create a seal around the treatment zone when the aforementioned shut-off part is placed in contact with the treatment head while the device is not being used for treating the human keratinous material.

The flexible lip may allow air to enter the treatment zone, notably if the suction generated by the pump becomes too great.

### Device

As a preference, the device is a non-therapeutic device, particularly for topical application of a cosmetic composition.

The device according to the invention allows effective treatment both of exogenic impurities and of endogenic impurities or defects, while at the same time meeting certain currently applicable environmental requirements and safety standards.

At least part of the device may heat and/or diffuse light and/or cool, thereby for example making it possible to activate certain compounds present in the fluid, notably in the cosmetic composition.

The device, notably the handpiece and/or the support, may at least partially vibrate, thereby notably making it possible to dislodge certain impurities stuck in the reservoir and/or the fluid circulation circuit, for example in the pump.

The device may be associated with additional grip means, for example for additional hands, these being arranged in such a way as to allow certain individuals living with disabilities to use it.

The device may comprise inscriptions in Braille, notably on certain parts of the handpiece, of the support, and/or of an external charger of the device.

### Cleaning and disinfection control

The device may comprise an electronic circuit driving the operation of the transducer and possibly of other elements of the device such as the electric pump and/or the UV radiation source.

The electronic circuit preferably comprises a control unit, for example involving a microcontroller, which may be borne by the handpiece and/or split between the support and the handpiece, or present on the support only.

The device may comprise several sensors, such as a sensor sensing contact of the treatment head with the keratinous material that is to be treated, or with the shut-off part, or a sensor that senses the fill or presence of the reservoir, which sensors are able to communicate data to the control unit. The device may comprise at least one sensor preventing UV from being emitted as long as predefined conditions are not met, for example conditions that guarantee that UV radiation cannot escape towards the user during the decontamination operation, for example sensing that the handpiece is correctly positioned on its support.

The device may further comprise at least one human-machine interface communicating with the control unit, notably on the handpiece and/or the support. The human-machine interface may comprise a display screen and/or control buttons.

The interface may offer various functions and/or programs making it possible for example to set certain operating parameters of the device, or indicate certain information to the user, such as the battery level, the time of use, the liquid level in the reservoir, the level of fouling of the various washable elements (reservoir, filters, etc.), user-specific information or else information relating to the safety and/or conditions of use of the device.

The interface may enable the selection of a mode of operation for treating the keratinous material and a cleaning and decontamination mode in which at least one UV source is switched on.

The interface may communicate via a wired or wireless connection, for example over Wi-Fi or Bluetooth, with a terminal such as a mobile phone, for example so as to receive updates or transmit data associated with the use of the device.

The interface may further comprise functionalities enabling diagnostics of the keratinous material that is to be treated.

The device may comprise various switches, notably an on/off and/or standby switch, a switch for selecting a program or selecting certain parameters specific to the ultrasound waves, enabling selection of the pump throughput or a device self-cleaning mode.

The device may even comprise one or more LED-type light sources which may be used to illuminate the zone that is to be treated or else as indicators, for example notifying the user of the need to change, fill or empty the reservoir or to clean the filtration system, or indicating whether the UV source or sources are in the active or inactive state and/or indicating the mode of operation of the device, for example the keratinous material treatment mode or the self-cleaning mode.

### Fluid

The fluid, notably the cosmetic composition, may comprise at least one surfactant.

The fluid, notably the cosmetic composition, may have a total concentration of surfactant(s) of between 0.01% and 20% by weight with respect to the total mass of the fluid, and notably of the cosmetic composition, better still between 0.01% and 5%, even better still 0.1% and 1.5%.

The cosmetic composition may or may not have bubbles already present within it before the acoustic waves are applied.

### Cleaning and disinfection method

A further subject of the invention, according to another of the aspects of the invention, is a method for cleaning and disinfecting a device for treating keratinous material comprising at least one reservoir for containing a fluid and/or one fluid circulation circuit, and at least one UV radiation source, notably a device for treating keratinous material as defined hereinabove according to the first aspect of the invention, the method comprising:
(1) emitting UV radiation from said at least one UV radiation source onto at least part of an internal surface of said at least one reservoir and/or of the fluid circulation circuit, and optionally towards a treatment head of the device.

If the device comprises a handpiece containing the reservoir, and a support onto which the handpiece can be set down, then the method may comprise, prior to step (1):
(2) positioning the handpiece on the support.

The method may comprise:
(3) detaching the reservoir from the handpiece,
(4) positioning the now-detached reservoir on the support,
(5) emitting UV radiation into the reservoir.

The method may comprise detaching the treatment head from the handpiece and positioning the detached treatment head on the support, then emitting UV radiation onto the treatment head.

If the device comprises a fluid circulation circuit, the method may comprise reflecting at least some of the UV radiation off surfaces of the fluid circulation circuit and/or off interior and/or exterior surfaces of the reservoir, and/or off interior surfaces of the handpiece.

If the handpiece comprises a treatment head defining a treatment zone and a fluid circulation circuit with a canal for conveying the fluid to the treatment zone and a canal for returning the fluid from the treatment zone, and if also the support comprises a shut-off part, the method may comprise:
(6) positioning the handpiece on the support in such a way that the shut-off part comes into contact with the handpiece and closes off the treatment zone,
(7) recirculating the fluid in the fluid circulation circuit.

During step (7), recirculation can be performed by a pump present in the handpiece and electrically powered by the support, for example by virtue of an electrical connection between the handpiece and the support or by the inductive transmission of electrical power between the support and the handpiece.

During step (7), an ultrasound transducer present in the handpiece may be activated in order to generate cavitation and enhance the cleaning action.

This transducer may be electrically powered by the support.

During step (7), the fluid may be exposed to the UV radiation, as may surfaces of the treatment zone and/or of the reservoir.

A further subject of the invention, according to another of the aspects of the invention, is a method for cleaning and disinfecting a device for treating keratinous material, notably a device according to the first or second aspect of the invention, as defined above, the method comprising:
(1) positioning the handpiece on the support in such a way that the shut-off part comes into contact with the handpiece and closes off the treatment zone,
(2) recirculating the fluid in the fluid circulation circuit.

The support may power the handpiece when the latter is positioned on the support.

The method may comprise:
(3) detaching said at least one reservoir from the handpiece,
(4) positioning the now-detached reservoir on the support.

During the recirculation of the liquid while the shut-off part is closing the treatment zone, the UV radiation source or sources may be in operation, as may the ultrasound transducer. The fluid may be exposed to the UV radiation as it is being recirculated.

The fluid that circulates during the cleaning phase may be water, a cleaning composition containing at least one active cleaning agent, or the cosmetic composition used elsewhere in the treatment of the keratinous material.

The cleaning and disinfection method may be performed before and/or after use of the device for treating the keratinous material.

### Brief description of the drawings

The invention may be understood more clearly on reading the following detailed description of nonlimiting implementation examples thereof and on examining the appended drawing, in which:
[Fig 1] schematically and partially illustrates one example of a device according to the invention,
[Fig 2] schematically and partially illustrates the handpiece of the device of Figure 1,
[Fig 3A] schematically and partially illustrates the device of Figure 1 with the reservoir detached from the handpiece,
[Fig 3B] is a schematic and partial cross section of the support of the device of Figure 1 with the reservoir detached from the handpiece,
[Fig 4] schematically and partially illustrates the device of Figure 1 in the form of a block diagram,
[Fig 5] schematically and partially illustrates a first example of a method for cleaning and disinfecting the device of Figure 1,
[Fig 6A] schematically and partially illustrates the support of the device of Figure 1, with UV lamps,
[Fig 6B] schematically and partially illustrates a second example of a method for cleaning and disinfecting the device of Figure 1,
[Fig 7] schematically and partially illustrates an environment in which the device of Figure 1 is used,
[Fig 8] is a schematic and partial cross section of the handpiece of a second example of a device according to the invention, and
[Fig 9] is a schematic and partial cross section of the handpiece of a third example of a device according to the invention.

### Detailed description

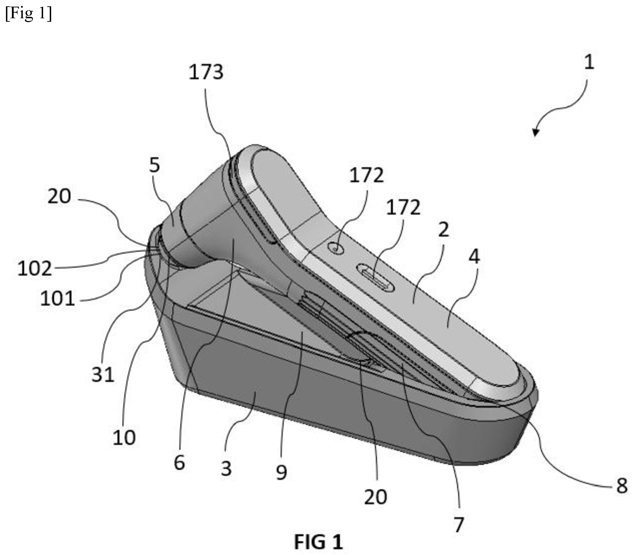

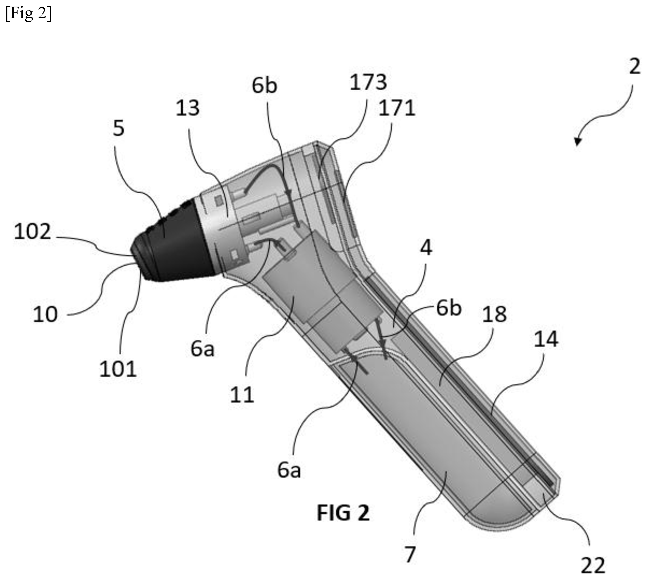

Figures 1 to 7 illustrates a device 1 for treating the skin, according to the invention, comprising a handpiece 2 and a support 3.

### Handpiece

The handpiece 2 comprises a handle 4, a treatment head 5, a circuit 6 for circulating a composition C and at least one reservoir 7 containing this composition C.

The reservoir 7 makes it possible, during the operation of the device, to compensate for any losses of composition, in the event that a portion thereof is not recycled.

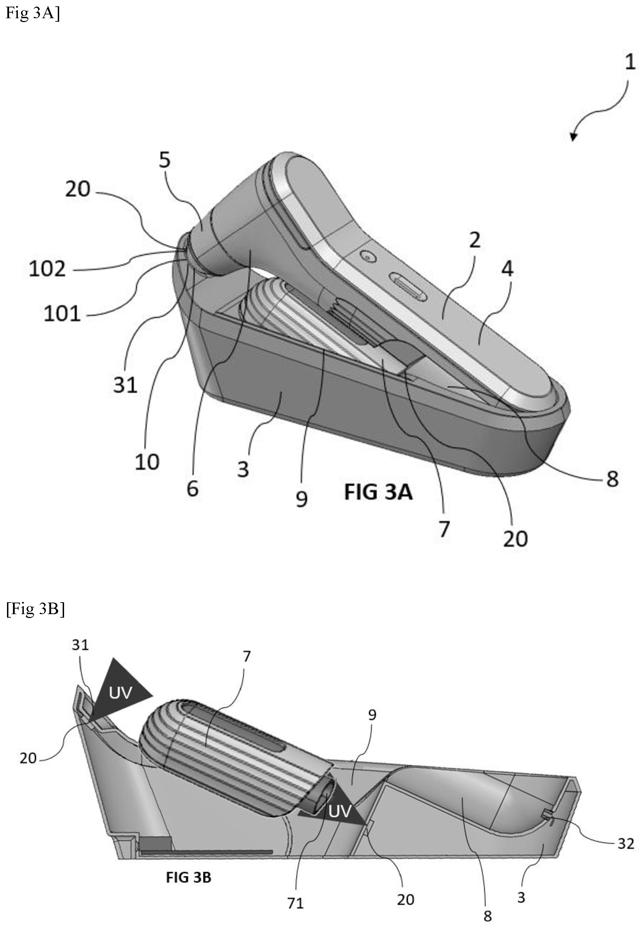

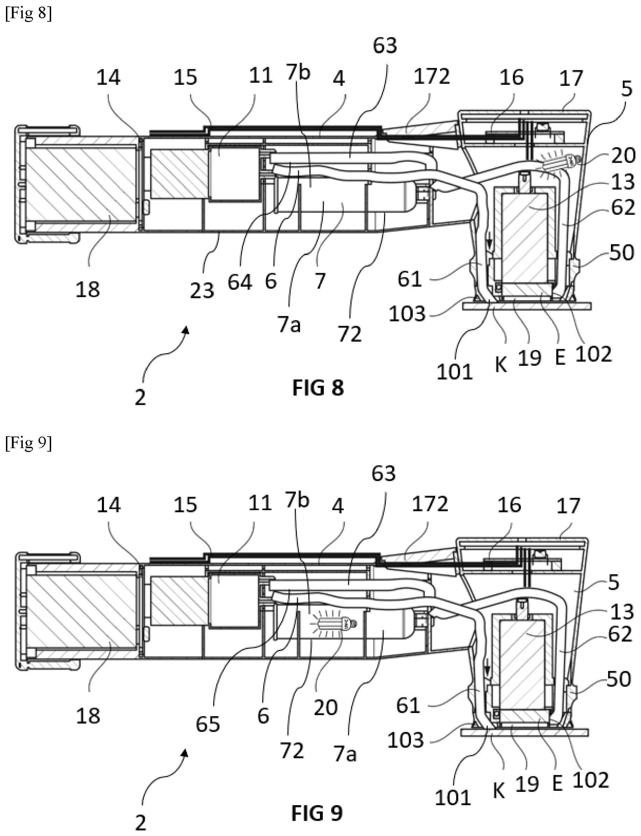

The reservoir 7 is preferably detachable from the handpiece, as illustrated in Figures 3A and 3B. In a variant, the reservoir 7 is not detachable from the handpiece, as in the variants illustrated in Figures 8 and 9.

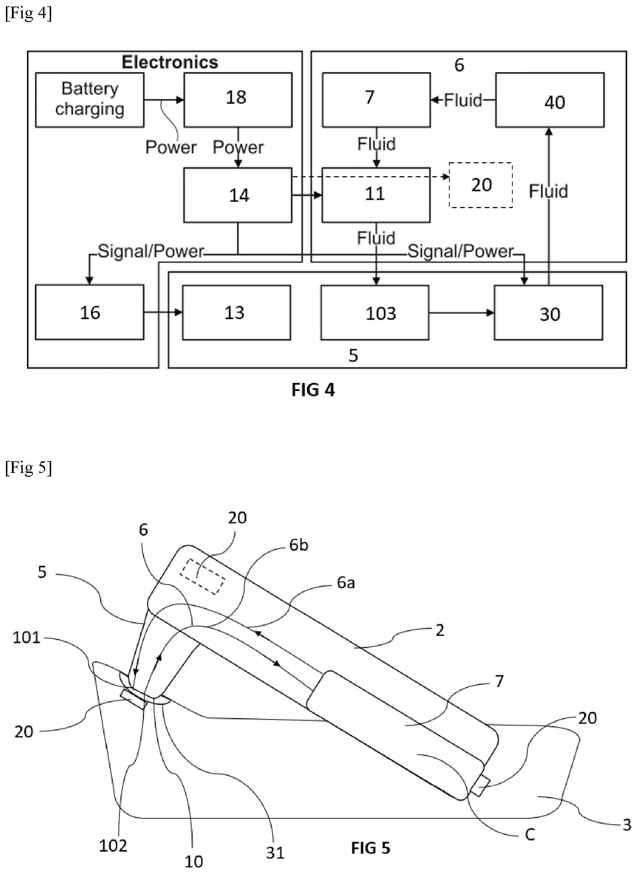

The circuit 6 comprises a pump 11 schematically depicted in Figure 5. The pump 11 may have any type of drive, notably manual or electric, and preferably electric. It may be of varying type. The pump 11 is for example a positive-displacement pump or centrifugal pump, notably a peristaltic pump, diaphragm pump, or piston pump, or combination of several types of pump. The pump 11 may be powered by direct current or alternating current. As a preference, the power supply voltage of the pump 11 is less than 50 V, being for example powered at a voltage ranging from 5 to 12 V. The pump 11 is preferably compatible with liquids of varying nature, notably aqueous, oily, mixed liquids containing particles or fillers in suspension, or even polymers. The mechanism of the pump 11 may be driven as direct drive from a motor or via a reduction gear. When the drive is a manual drive, the device 1 may comprise a handle that the user actuates in order to operate the pump 11. As a preference, the pump 11 is configured to provide a fluid flow rate of between 0.01 ml and 15 ml per second. The pump 11 may or may not be removable. The pump 11 may generate a negative and/or positive pressure on the fluid in order to cause the fluid to circulate and bring it into contact with the skin. The pump may also be used for the internal cleaning of all or part of the device. The pump 11 may contain a single-part or multi-part filter. The pump 11 can be mechanically or electrically isolated in the device 1, for example so that it can be removed from the device 1 for cleaning.

The treatment head 5 comprises a surface 10 in contact with the composition C, the treatment head 5 being designed to deliver the composition C to a zone of the keratinous material K that is to be treated. The composition leaves the treatment head 5 via a first opening 101 of a duct 6a. The handpiece 2 can be moved along the zone that is to be treated and it is possible for the composition C that is delivered by the opening 101 not to be recycled. As a variant, recycling of the composition C is performed, the composition leaving via the opening 101 recirculating in the circuit 6 and optionally the reservoir 7 after having been reinjected into the circuit 6 via a second opening 102 of a duct 6b present on the treatment head 5.

The duct 6b may communicate with a suction pump 30, for example an electric pump, which may send the delivered composition C into a treatment and purification unit 40 that enables the composition C to be recycled so that it can be sent back to the keratinous material K that is to be treated, the composition C then circulating at least partially in closed-circuit. The treatment and purification unit is, for example, a filtration system comprising one or more filters designed to hold back particles in suspension in the composition, such as skin debris removed during cleansing. A filtration system is for example arranged between the suction pump and the reservoir 7 so that the composition C is returned to the reservoir 7 after filtration.

The handpiece may, where applicable, comprise, around the openings 101 and 102 of the treatment head 5, a sealing element 103 depicted schematically in Figure 5, such as a flexible lip, in order to contain the composition C when the head is applied to the skin, and facilitate its return via the duct 6b. The lip may be made up of sub-elements between which the composition can be recovered.

The treatment head 5 comprises at least one transducer 13 emitting acoustic waves and a sonotrode, the surface 10 in contact with the composition being defined for example by a free end of the sonotrode. Bubbles may be generated in the composition C as a result of the acoustic waves owing to the power and frequency of the acoustic waves.

The transducer 13 or the sonotrode thereof are preferably removable. Thus, these can easily be removed from the device in order to clean them.

The emission surface 10 for emitting the acoustic waves towards the liquid environment may comprise reliefs or etchings from which the bubbles may originate at different levels along the longitudinal axis of the transducer 13. In particular, these reliefs may offer nucleation sites at their tip and at their base. The reliefs may allow a more uniform production of bubbles. These reliefs may also encourage the collapse of the bubbles because the differences in level along the longitudinal axis make it possible to generate relatively extensive cavitation zones that are not restricted to a single plane. In addition to the reliefs on the emission surface 10, which contribute to improving the production of bubbles, the emission surface may have reliefs that pursue another goal, for example that of retaining the fluid or of cleaning the zone that is to be treated. These for example are reliefs made of a flexible material, for example bristles, notably flocking.

The handpiece 2 may further comprise an electronic circuit 14.

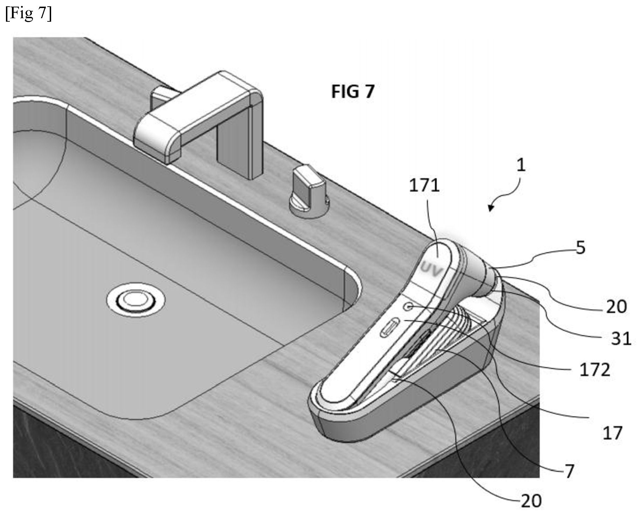

The electronic circuit 14 may comprise a control unit able to communicate with a human-machine interface 17, notably visible in Figure 7, it being possible for the interface 17 to comprise a grille 171 and/or control buttons 172 and/or indicator lamps 173, or even to communicate wirelessly with a terminal such as a mobile phone. The indicator lamps 173 are able to inform the user as to the state of operation of the device and/or as to the mode of operation thereof, notably through the illumination and/or flashing of coloured LEDs.

The human-machine interface 17 notably enables the device to be diagnosed remotely, to be reprogrammed, to receive software updates and/or to automatically issue orders for the delivery of accessories to replace spent accessories, cleaning and/or treatment products.

The human-machine interface 17 may make it possible to adjust certain operating parameters of the device 1, for example the intensity with which the acoustic waves are emitted.

The electronic circuit 14 is able to drive the operation of the pump 11, of the suction pump 30, of a battery 18 powering the device (notably the state of charge thereof) and to receive data from one or more sensors (not depicted) such as a sensor sensing contact of the device with the keratinous material.

The electronic circuit 14 may also communicate with an optical detector, not depicted, able to detect the presence of a compound that is to be removed from the surface of the keratinous material, and possibly to determine the quantity present.

The electronic circuit 14 is also able to detect when the handpiece 2 is held on the support 3 and therefore start the pump to begin to circulate the composition C in closed-loop in the handpiece 2, allowing cleaning and disinfection of the circuit 6 and of the reservoir 7, as illustrated in Figure 5.

The electronic circuit 14 is connected by connections 15 to an electronic generator 16 notably powering the transducer 13. This generator 16 comprises for example an oscillator and a power stage.

The electronic circuit 14 can start the pump 11 and/or the suction pump 30 and/or the emission of the acoustic waves by the transducer 13 only when the treatment zone is in contact with the skin or with a shut-off part, notably in such a way as to allow the composition to be recycled.

The battery 18 may be removable; it may be used for powering the entirety of the device 1, or just part, notably the pump 11. The battery 18 may be of varying type, for example Li-ion or NiMH, preferably of a type suited to wet conditions or which can be at least partially immersed in a liquid. The battery 18 is preferably rechargeable. The battery 18 is preferably selected to be able, without needing to be recharged, to power the device 1 for a number of consecutive treatment sessions, or even several treatment sessions and one cleaning of the device 1.

### Support

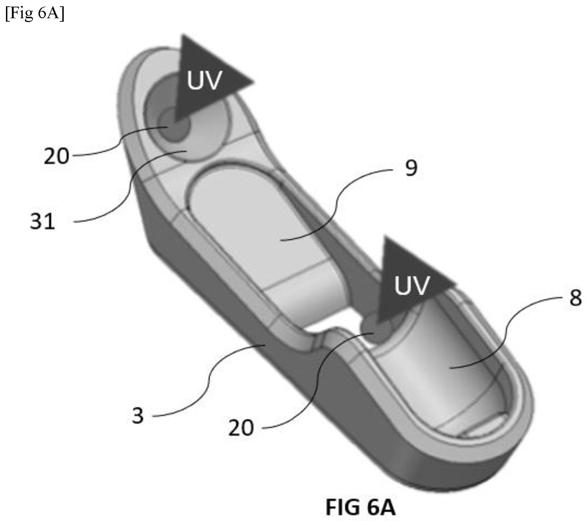

The support 3 comprises, as illustrated in Figure 6A, a first housing 8 to accept the handpiece 2, and a second housing 9 to accept the reservoir with an opening 71 of the reservoir 7 oriented downwards.

The reservoir 7 may be accommodated in the housing 9 of the support, as visible in Figures 3A and 3B, while the handpiece 2 detached from the reservoir 7 is accommodated in the housing 8 of the support 3.

The support 3 comprises a shut-off part 31 designed to collaborate with the surface 10 of the treatment head 5 in order to close off the fluidic circuit while still for example allowing the fluid to circulate in the treatment zone.

The device is for example charged via a USB port (not depicted) notably sited on the support 3. The support 3 comprises, for example, as illustrated, a first connector 32 and the handpiece 2 a second connector 22 which is designed to connect with the first connector 32 when the handpiece 2 is held on the support 3, this connection enabling the handpiece 2 to be charged. The connectors 22 and 32 may be connectors at least one of which is spring-loaded.

In a variant, the handpiece 2 may be charged by induction when held on the support 3.

The battery 18 may also be removed and charged separately.

The device 1 comprises at least one UV radiation source 20, for example one or more UV-C lamps, enabling the internal and external surfaces of the device to be cleaned and disinfected.

The UV radiation source 20 allows disinfection of all or part of the internal and/or external parts of the device 1, notably of the reservoir 7, of the circuit 6, of the treatment and purification units 40, of the pumps 11 and 30 and/or of the surface or surfaces of the device in contact with the keratinous material.

The surfaces in contact with the keratinous material are for example the treatment head 5, a grille and/or a guiding and/or combing member.

Each UV radiation source is able to emit radiation of a wavelength comprised between 100 nm and 400 nm, preferably between 100 nm and 280 nm (UV-C radiation), notably between 250 nm and 270 nm, for example equal to 254 nm, 265 nm or 185 nm. The UV radiation source may comprise a UV-C LED, thereby enabling energy savings and a long lifetime of the source. The UV radiation source may emit UV radiation of an intensity I of between 2.9 mW/cm² and 1080 mW/cm².

The UV radiation may pass fully or partially through the materials of which the device is made and/or through any liquid and/or other component stored and/or transported in the device. The UV radiation may be completely or partially reflected by materials of which the device is made.

As illustrated in Figures 4 and 5, UV-C radiation sources 20 may be found on the handpiece 2, for example in order to expose part of the fluid circulation circuit and/or of the reservoir to the UV radiation.

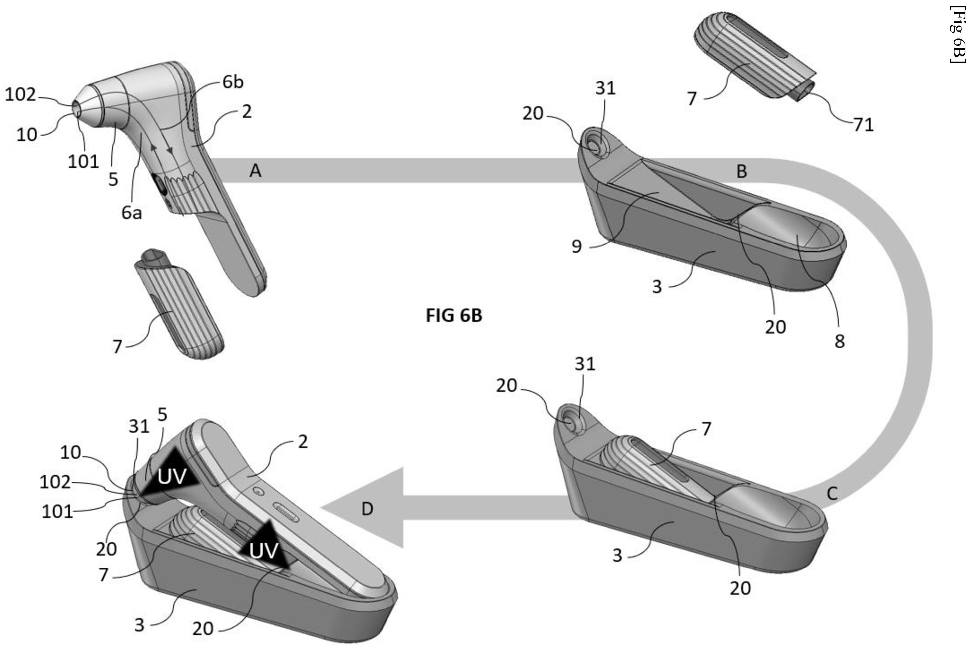

As illustrated in Figures 6A and 6B, the UV-C lamps 20 may be situated on the support 3. These UV-C lamps 20 are, for example, situated in the region of the shut-off part 31, so as to disinfect the surface 10 of the treatment head 5 and the openings 101 and 102, and in the region of the housing 9 near the opening 71 of the reservoir 7 when held in the housing 9, so as to disinfect this reservoir.

The UV-C lamps 20 are for example OSRAM-brand OSLON^{®} UV 3535, SU CULBN2.VC LEDs having an emission peak at 275 nm. This model of lamp has the advantage of being compact and economical.

### Use for the treatment of keratinous material

The device 1 may be used for the treatment of keratinous material K, for example the skin.

The treatment head 5 is thus brought into contact with the keratinous material K by the user manipulating the handpiece 2, and this user may switch the device 1 on while the treatment head is pressed against the keratinous material. The composition C coming from the reservoir 7 then circulates in the circuit 6, notably in the duct 6a, through the driving of the pump 11. The composition C circulating in the duct 6a is released into contact with the keratinous material K through the opening 101.

The transducer 13 generates acoustic waves enabling the formation of bubbles within the cosmetic composition C and the collapse of said bubbles, thus enabling the desired treatment of the keratinous material K.

The composition C is, at least partly, reinjected into the circuit 6, notably by virtue of the suction pump 30. The composition C re-enters the treatment head 5 via the opening 102 to be sent to the treatment and purification unit 40.

On leaving the treatment and purification unit 40, the composition C is sent back to the reservoir 7.

### Cleaning and disinfection

After use, the device may be cleaned by implementing one of the methods illustrated in Figures 5 or 6B, or both methods one after the other.

In Figure 5, the handpiece 2 is held on the support 3 with the reservoir 7 not detached. The liquid contained in the reservoir is made to circulate by switching on the pump 11. The shut-off part 31 closes off the treatment zone by virtue of the sealing element 103, so that the composition circulating in the duct 6a and leaving via the opening 101 returns to the circuit 6 and the reservoir 7 after having been reinjected into the circuit 6 via the opening 102, this being achieved by virtue of the suction of the pump 30.

During this cleaning, the reservoir 7 may be prefilled with a disinfectant and/or cleaning composition in order to improve the cleaning and the disinfection of the circuit 6 and of the reservoir 7.

In Figure 6B, in step A, the reservoir 7 is detached from the handpiece 2. It is then positioned in the housing 9 of the support 3 in step B, with the opening 71 of the reservoir 7 directed downwards. In step C, the reservoir 7 is drained in the housing 9. Finally, in step D, the handpiece 2 is held in the housing 8 of the support 3 with the surface 10 of the treatment head 5 facing the shut-off part 31 and the UV radiation sources 20 are switched on in order to disinfect the reservoir 7 and the surface 10 as well as the openings 101 and 102 present on the treatment head, and the circuit 6.

In a variant which has not been illustrated, the reservoir 7 is not detached from the handpiece 2 and the latter is held in the housing 8 of the support 3 with the surface 10 of the treatment head 5 facing the shut-off part 31. The method described in Figure 5 is then implemented and the UV radiation sources 20 of the support 3, as illustrated in Figure 6A, are also switched on. The reservoir 7 is for example made from a UV-transparent material so that the UV radiation can contribute to disinfecting it.

In another variant which has not been illustrated, the support 3 comprises a single UV radiation source situated at the shut-off part 31. The reservoir 7 is not detached from the handpiece 2 and the latter is held in the housing 8 of the support 3 with the surface 10 of the treatment head 5 facing the shut-off part 31. The method described in Figure 5 is then implemented and the UV radiation source is switched on.

### Embodiment variants

A variant of the device 1 is depicted in Figures 8 and 9. This device comprises a handpiece 2 comprising two reservoirs 7a and 7b that cannot be detached from the handpiece 2 and comprises no support 3.

The handpiece 2 is designed to distribute the composition C to a zone of keratinous material K that is to be treated, via at least one opening 101 situated at the treatment head 5. The reservoir 7a is, for example, a reservoir of composition to be distributed; it is connected to the pump 11 via a duct 63 which may, as illustrated, convey the composition C to the treatment zone via a duct 61 which opens out via the opening 101. The reservoir 7b recovers the composition C that has been in contact with the zone that is to be treated by means of a duct 62 which opens out onto an opening 102.

In the devices of Figures 8 and 9, the UV-C lamps 20 are situated inside the handpiece 2, respectively in the treatment head 5 and inside the reservoir 7.

In the example of Figure 8, the wall 23 of the handpiece 2 may be covered on the inside with aluminium, notably aluminium foil, able to reflect the UV radiation so as to concentrate it inside the handpiece with a view to obtaining better disinfection of the elements present inside said handpiece. The walls 72 of the reservoir 7 and the walls 64 of the circuit 6 are for example made of FEP which is a plastics material able to transmit the UV radiation into the circuit 6 and into the reservoir 7.

In the example of Figure 9, the wall 72 of the reservoir and the wall of the circuit 6 may be covered on the inside with aluminium, notably aluminium foil, so as to concentrate the UV radiation inside the reservoir 7 and inside the circuit 6 for a better disinfection of those components. The UV lamp 20 also enables the disinfection of the composition C circulating in the circuit 6.

The treatment head 5 in this example comprises a transducer 13 arranged relative to a spacer 50 so as to create a space E between the acoustic waves emission surface and the surface of the keratinous material K that is to be treated.

The space 50 advantageously comprises at least one leg extending axially over less than 360° about the axis of the transducer 13, and in front of the emission surface. What is to be understood by "in front of" is that the leg extends beyond the emission surface in the direction of the keratinous material K that is to be treated. The spacer 50 may be in the form of an endpiece attached to the rest of the treatment head 5 by means of a fixing part, or may be of one-piece with the body of this head, for example.

The treatment head 5 may, as illustrated, comprise a grille 19, preferably at least partially non-absorbing, defining a surface of contact with the keratinous material K that is to be treated. The grille 19 has at least one opening through which the waves emitted by the transducer 13 are able to travel towards the material K that is to be cleansed. Thanks to the grille 19, it is possible to exercise more precise control over the distance between the transducer 13 and the surface of said keratinous material K, thereby making it possible to improve the action of the bubbles near the surface of the keratinous material K while at the same time avoiding direct contact of the transducer 13 with this keratinous material K. The grille 19 is preferably removable. That makes it easier to clean and allows it to be replaced between uses, or even, if so desired, allows several grilles to be interchanged according to the use, for example by selecting the kind of grille best suited to the morphology of the zone that is to be cleansed.

The device 1 may comprise at least one fluid circulation duct extending along the leg or legs or within these, or more generally in the part supporting the grille and/or in the grille. This duct may be made from a UV-transparent material.

Of course, the invention is not limited to the examples that have just been described. The support 3 and the handpiece 2 of the one same device 1 may notably both comprise at least one UV radiation source 20.

The device may comprise a bubble generator for generating additional bubbles within the fluid. The bubble generator may be arranged in the device 1 in such a way as to generate bubbles prior to, simultaneously with, or cyclically in relation to the emission of the acoustic waves.

In the case of a hair treatment, the device according to the invention may comprise at least one guiding and/or combing member designed to guide the hair as it moves close to and/or into contact with the emission surface of the transducer. The guiding and/or combing member may be designed to guide the hair laterally, for example having end stops between which the hair is engaged. The guiding and/or combing member may comprise at least one lateral hair-guiding surface enabling the hair to be held in position as it is being treated. The guiding and/or combing member may be attached to the device removably or non-removably.

It is possible for the handpiece to comprise just one single pump which circulates the fluid in the treatment zone and returns it to a reservoir intended to recover the used fluid.

## Claims

1. Device (1) for treating human keratinous material (K), notably the skin and/or the hair, comprising:
- a treatment head (5) to be placed in contact with the keratinous material (K) that is to be treated, defining at least one treatment zone,
- at least one reservoir (7) for containing a fluid, notably a cosmetic composition (C) used in the treatment, this reservoir (7) having an internal surface in contact with the fluid,
- at least one source (20) of UV radiation,
- at least one fluid circulation circuit (6) connecting the reservoir (7) and the treatment zone, at least part of the internal surface of the reservoir (7) and/or of the fluid circulation circuit (6) being exposed to the UV radiation emitted by the source (20).

2. Device (1) according to the preceding claim comprising :
at least one ultrasound transducer (13) for emitting acoustic waves at least into the fluid present in the treatment zone.

3. Device according to any one of claims 1 and 2, comprising a handpiece (2) comprising the treatment head (5), the reservoir or reservoirs (7), the ultrasound transducer or transducers (13) and/or the fluid circulation circuit (6), and the device comprising a support (3) designed to accept the handpiece (2) when the device is not being used for the treatment of the keratinous material (K).

4. Device according to the preceding claim, the support (3) comprising at least one UV radiation source (20), notably one or two.

5. Device according to any one of the preceding claims, wherein said at least one UV radiation source (20) is situated inside the handpiece (2).

6. Device according to any one of the preceding claims, the support (3) comprising at least one UV radiation source (20) arranged to emit UV radiation inside the reservoir (7) and/or at least one UV radiation source (20) arranged to emit UV radiation onto the treatment head (5).

7. Device according to any one of the preceding claims, the fluid circulation circuit (6) comprising at least an electric pump (11) for circulating the fluid in the fluid circulation circuit between the reservoir or reservoirs (7) and the treatment zone and/or the fluid circulation circuit (6) comprising at least one UV radiation source (20) and/or at least one reservoir (7) of the device comprising at least one UV radiation source (20) that is situated inside the reservoir (7).

8. Device according to any one of the preceding Claims, the reservoir (7) being removable, the support (3) preferably comprising a housing (9) designed to accept the reservoir (7) detached from the handpiece (2), notably designed to accept the reservoir (7) with an opening (71) of the reservoir (7) being directed downwards.

9. Device according to any one of the preceding Claims, the device, notably the handpiece (2), comprising a material that reflects over 50% of the UV, notably UVC, radiation, for example aluminium.

10. Device according to any one of Claims 1 to 9, at least part of an internal surface of the device (1) being covered with a metal coating, notably an aluminium coating, to enable propagation of the UV radiation and/or at least part of a duct (6a; 6b; 61; 62; 63) of the fluid circulation circuit (6) being covered with a metal coating, notably an aluminium coating, to encourage propagation of the UV radiation within the fluid circulation circuit (6) and/or towards the reservoir (7).

11. Device according to any one of the preceding Claims in combination with claim 2, the transducer (13) extending at least partially into the treatment head (5).

12. Device according to any one of the preceding Claims in combination with claim 3, the support (3) being designed to electrically power the handpiece (2), notably the pump or pumps (11) comprised within the handpiece (2) when the handpiece (2) is held in the support (3).

13. Method for cleaning and disinfecting a device (1) for treating keratinous material (K) comprising at least one reservoir (7) for containing a fluid and at least one UV radiation source (20), notably a device (1) for treating keratinous material (K) as defined in any one of Claims 1 to 12, the method comprising:
(1) emitting UV radiation from said at least one UV radiation source (20) onto at least part of an internal surface (72) of said at least one reservoir (7) and/or of a fluid circulation circuit, and optionally towards a treatment head (5) of the device (1),
wherein, when the device comprises a handpiece (2) comprising the reservoir (7), and a support (3), the method comprises, prior to step (1):
(2) positioning the handpiece (2) on the support (3).

14. Method according to the preceding claim, comprising :
(1) detaching the reservoir (7) from the handpiece (2),
(2) positioning the now-detached reservoir (7) on the support (3),
(3) emitting UV radiation into the reservoir (7),
wherein, when the device (1) comprises a fluid circulation circuit (6), the method comprises reflecting at least some of the UV radiation off surfaces (64) of the fluid circulation circuit and/or off interior and/or exterior surfaces (72) of the reservoir, and/or off interior surfaces of the handpiece (2),

15. Method according to any one of claims 13 and 14, wherein, when the handpiece (2) comprises a treatment head (5) defining a treatment zone and a fluid circulation circuit (6) with a canal (6a; 61) for conveying the fluid to the treatment zone and a canal (6b; 62) for returning the fluid from the treatment zone, and the support (3) comprising a shut-off part (31), the method comprises:
(1) positioning the handpiece (2) on the support (3) in such a way that the shut-off part (31) comes into contact with the handpiece (2) and closes off the treatment zone,
(2) recirculating the fluid in the fluid circulation circuit (6).
